(19) **Europäisches Patentamt / European Patent Office / Office européen des brevets**

(11) **EP 3 364 777 B1**

(12) **FASCICULE DE BREVET EUROPEEN**

(45) Date de publication et mention
de la délivrance du brevet:
**24.02.2021 Bulletin 2021/08**

(21) Numéro de dépôt: **16809457.1**

(22) Date de dépôt: **18.10.2016**

(51) Int Cl.:
*A23L 3/16* (2006.01)       *A23L 21/20* (2016.01)
*A23L 29/275* (2016.01)     *A23L 33/15* (2016.01)
*A23P 30/20* (2016.01)      *A23K 10/24* (2016.01)
*A23K 10/22* (2016.01)      *A23K 50/80* (2016.01)
*A23K 20/174* (2016.01)     *C08L 5/08* (2006.01)

(86) Numéro de dépôt international:
**PCT/FR2016/052694**

(87) Numéro de publication internationale:
**WO 2017/068278 (27.04.2017 Gazette 2017/17)**

(54) **PRESERVATION DE VITAMINES HYDROSOLUBLES**

KONSERVIERUNG VON WASSERLÖSLICHEN VITAMINEN

PRESERVATION OF WATER-SOLUBLE VITAMINS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.10.2015 FR 1560014**

(43) Date de publication de la demande:
**29.08.2018 Bulletin 2018/35**

(73) Titulaire: **Ynsect**
**91058 Évry-Courcouronnes Cedex (FR)**

(72) Inventeurs:
• **HUBERT, Antoine**
**94140 Alfortville (FR)**
• **BEREZINA, Nathalie**
**75005 Paris (FR)**
• **ARMENJON, Benjamin**
**75013 Paris (FR)**

(74) Mandataire: **Santarelli**
**49, avenue des Champs-Elysées**
**75008 Paris (FR)**

(56) Documents cités:
**EP-A2- 0 013 181      KR-A- 20110 013 563**

• **DATABASE WPI Week 201529 Thomson
Scientific, London, GB; AN 2015-25043R
XP002754229, & CN 104 382 059 A (ZHENG H) 4
mars 2015 (2015-03-04)**
• **DATABASE WPI Week 201071 Thomson
Scientific, London, GB; AN 2010-K69645
XP002754230, -& CN 101 779 723 A (NINGBO
TECH BANK CO LTD) 21 juillet 2010 (2010-07-21)**
• **Li-Ming Zhao ET AL: "Preparation and application
of chitosan nanoparticles and nanofibers",
Brazilian Journal of Chemical Engineering, 1
septembre 2011 (2011-09-01), pages 353-362,
XP055249623, DOI:
10.1590/S0104-66322011000300001 Extrait de
l'Internet:
URL:http://www.scielo.br/pdf/bjce/v28n3/v2
8n3a01.pdf [extrait le 2016-02-12]**
• **MARTIN-DIANA A B ET AL: "Orange juices
enriched with chitosan: Optimisation for
extending the shelf-life", INNOVATIVE FOOD
SCIENCE AND EMERGING TECHNOLOGIES,
ELSEVIER, AMSTERDAM, NL, vol. 10, no. 4, 1
octobre 2009 (2009-10-01), pages 590-600,
XP026501987, ISSN: 1466-8564, DOI:
10.1016/J.IFSET.2009.05.003 [extrait le
2009-05-12]**
• **KOIDE S S: "Chitin-chitosan: properties, benefits
and risks", NUTRITION RESEARCH, ELSEVIER
INC, XX, vol. 18, no. 6, 1 juin 1998 (1998-06-01),
pages 1091-1101, XP009161185, ISSN: 0271-5317**

EP 3 364 777 B1

**Description**

La présente invention se rapporte à un procédé comprenant un traitement thermique d'une composition comprenant des vitamines hydrosolubles. L'invention vise également les granulés obtenus par le procédé selon l'invention, et l'utilisation de ces granulés dans l'alimentation humaine ou animale, en particulier dans l'alimentation des poissons.

L'aquaculture est aujourd'hui l'un des secteurs les plus dynamiques de l'industrie alimentaire. La forte demande en poissons a conduit les chercheurs à développer des aliments aquacoles qui permettraient une culture efficace et rapide des poissons.

Généralement, les aliments aquacoles comprennent de la farine de poisson. En effet, la farine de poisson est une des principales sources de protéines dans ces aliments. C'est une farine qui est très riche en protéines animales (et en particulier riche en acides aminés de type lysine et méthionine) faciles à digérer.

Les aliments aquacoles peuvent également comprendre des vitamines. Il est bien connu que les vitamines, en particulier les vitamines hydrosolubles, ont un rôle important dans le développement et la croissance des poissons. Ainsi, il est courant d'ajouter des vitamines lors de la préparation des aliments aquacoles.

Les procédés de préparation d'aliments aquacoles, tel que l'extrusion, comprennent généralement une étape de traitement thermique à des températures supérieures ou égales à 90°C. Malheureusement, ce type de traitement thermique conduit à une dégradation des vitamines, en particulier des vitamines hydrosolubles, de sorte que les granulés obtenus ont une quantité réduite en vitamines.

La demande de brevet CN104382059 décrit un substitut de repas pour l'alimentation humaine.

La demande de brevet CN101779723 divulgue la préparation d'une composition pour nourrir des crevettes à pattes blanches.

Il existe donc un besoin pour des procédés de préparation d'aliments aquacoles, comprenant un traitement thermique, qui permettraient d'éviter ou limiter la dégradation des vitamines hydrosolubles au cours du traitement thermique.

Le travail des inventeurs a permis de mettre en évidence qu'il était possible de préserver les vitamines hydrosolubles lors d'un traitement thermique lorsque celui-ci était réalisé dans des conditions déterminées.

La présente invention concerne donc un procédé comprenant un traitement thermique d'une composition à une température supérieure ou égale à 90°C, dans lequel la composition comprend au moins 0,8% en poids de chitine et au moins 0,005% en poids de vitamines hydrosolubles et/ou leurs dérivés, par rapport au poids total de la composition.

On notera que dans le cadre de la présente demande, et sauf stipulation contraire, les gammes de valeurs indiquées s'entendent bornes incluses.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »). Ces copolymères peuvent également être associés à des pigments, souvent de type mélanine.

La chitine serait le deuxième polymère le plus synthétisé dans le monde vivant après la cellulose. En effet, la chitine est synthétisée par de nombreuses espèces du monde vivant: elle constitue en partie l'exosquelette des crustacés et des insectes et la paroi latérale qui entoure et protège les champignons. Plus particulièrement, chez les insectes, la chitine constitue ainsi 3 à 60% de leur exosquelette. La détermination du taux de chitine peut être effectuée par dosage suivant la méthode ADAC 991.43. Une telle méthode est utilisée dans l'Exemple 1, et représente une méthode préférée pour cette détermination.

La chitine présente dans la composition peut résulter de l'introduction dans la composition de chitine ou avantageusement de l'introduction dans la composition d'une farine d'arthropodes, de céphalopodes (comme le calmar), de gastéropodes, d'annélides, et/ou de champignons. Plus préférentiellement, la présence de chitine résulte de l'introduction d'une farine d'insectes, de crustacés, de calmar et/ou de champignons, encore plus préférentiellement d'une farine d'insectes et/ou de crustacés.

Encore plus préférentiellement, la chitine présente dans la composition résulte de l'introduction d'une farine d'insectes et optionnellement d'une farine de crustacés.

Par farine d'insectes, on vise plus particulièrement une poudre préparée à partir d'insectes. Une telle farine peut être préparée par un procédé comportant les étapes suivantes :

1. abattage des insectes,
2. pressage à chaud ou à froid des insectes pour obtenir un gâteau de presse,
3. séchage du gâteau de presse, et
4. broyage du gâteau de presse séché.

Ce procédé de préparation de la farine d'insectes est plus amplement détaillé ci-après.

Etape 1 : abattage des insectes

Cette étape 1 d'abattage peut avantageusement s'effectuer par ébouillantage ou par blanchiment. Cette étape 1 permet d'abattre les insectes tout en abaissant la charge microbienne (réduction du risque d'altération et sanitaire) et en inactivant les enzymes internes des insectes pouvant déclencher une autolyse, et ainsi un brunissement rapide de ceux-ci.

Pour l'ébouillantage, les insectes, de préférence des larves, sont ainsi ébouillantés à l'eau pendant 2 à 20 min, préférentiellement, 5 à 15 min. De préférence, l'eau est à température comprise entre 95 à 105°C, préférentiellement 100°C.

La quantité d'eau introduite lors de l'ébouillantage est déterminée de la façon suivante : le ratio du volume d'eau en mL sur le poids en g d'insecte est avantageusement compris entre 0,3 et 10, de préférence entre 0,5 et 5, plus préférentiellement entre 0,7 et 3, encore plus préférentiellement de l'ordre de 1.

Pour le blanchiment, les insectes, de préférence des larves, sont blanchis à la vapeur (buses ou lit de vapeur) à une température comprise entre 95 et 105°C, préférentiellement 98°C ou bien à l'eau à une température comprise entre 95 et 105°C, préférentiellement 100°C (par buses d'aspersion) ou en mode mixte (eau + vapeur). Le temps de séjour dans la chambre de blanchiment est compris entre 1 à 15 minutes, préférentiellement entre 3 et 7 min.

Etape 2 : pressage

Les insectes issus de l'étape 1 d'abattage sont ensuite placés dans une presse selon un mode opératoire qui permet de presser et séparer un jus comportant à la fois une fraction huileuse et une fraction protéique.

De préférence, l'étape de pressage permet d'obtenir un gâteau de presse comportant une teneur en huile inférieure ou égale à 20%, préférentiellement inférieure ou égale à 17%, plus préférentiellement inférieure ou égale à 15%, les pourcentages en poids étant donnés sur le poids sec de gâteau de presse.

De même, l'étape de pressage permet d'obtenir un gâteau de presse présentant une teneur en matière sèche comprise entre 30% et 60%, préférentiellement, comprise entre 40% et 55%, et plus préférentiellement comprise entre 45% et 55%, les pourcentages en poids étant donnés sur le poids de gâteau de presse.

Tout système de presse peut être utilisé pour réaliser l'étape de pressage, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est à même de déterminer les conditions de pressage afin d'obtenir les teneurs en huile et/ou en eau mentionnées ci-avant.

En particulier, il est possible d'effectuer un pressage à chaud ou à froid. Avantageusement, le pressage sera effectué à chaud, ce qui permet d'augmenter le déshuilage du gâteau de presse. En particulier, un pressage à chaud permet l'obtention d'un gâteau de presse comportant une teneur en huile inférieure ou égale à 17%, préférentiellement inférieure ou égale à 15%, les pourcentages en poids étant donnés sur le poids sec de gâteau de presse.

Etape 3 : séchage

Le gâteau de presse est ensuite séché par les technologies classiques connues de l'homme du métier. Le séchage peut être direct ou indirect (sécheur en couche mince, « paddle dryer », « tubular dryer », « disc-dryer », etc.) à une température comprise entre 60°C et 200°C, pendant une durée de 15 min à 24 heures. A titre d'exemple, le gâteau de presse peut être disposé et séché à l'air ventilé/brassé à une température comprise entre 80 et 100°C, préférentiellement à 90°C pendant une durée comprise entre 3 et 7 heures, préférentiellement 5 heures.

L'objectif de cette étape de séchage est l'obtention d'un gâteau de presse ayant un taux d'humidité compris entre 2 et 15%, de préférence entre 5 et 10%, plus préférentiellement encore entre 4 et 8%.

Etape 4 : broyage final

Le gâteau de presse séché est ensuite placé dans un broyeur, tel qu'un broyeur à marteaux, permettant de réduire le gâteau de presse en particules.

De préférence, à l'issue de ce broyage final, la taille des particules d'insectes est inférieure à 0,5 cm (plus grande taille de particule observable à l'aide d'un microscope), de préférence de l'ordre de 1 mm.

Les insectes préférés pour la préparation d'une telle farine sont par exemple les coléoptères, les diptères, les lépidoptères, les isoptères, les orthoptères, les hyménoptères, les blattoptères, les hémyptères, les hétéroptères, les éphéméroptères et les mécoptères, de préférence, les coléoptères, les diptères, les orthoptères et les lépidoptères.

Préférentiellement, les insectes sont choisis parmi le groupe constitué par *Tenebrio molitor, Hermetia illucens, Galleria mellonella, Alphitobius diaperinus, Zophobas morio, Blabera fusca, Tribolium castaneum, Rhynchophorus ferrugineus, Musca domestica, Chrysomya megacephala, Locusta migratoria, Schistocerca gregaria, Acheta domestica* et *Samia ricini,* et plus préférentiellement encore, *T. molitor.*

Avantageusement, la teneur en protéines de la farine d'insecte(s) est élevée, par exemple, supérieure à 67% en poids, préférentiellement supérieure à 68% en poids, encore plus préférentiellement supérieure à 70% en poids sur le poids total de farine d'insecte(s). Par « protéines », on vise la quantité de protéines brutes. La quantification des protéines brutes est bien connue de l'homme du métier. A titre d'exemple, on peut citer la méthode Dumas.

Par « vitamines hydrosolubles », on entend une ou plusieurs vitamines solubles dans l'eau, qu'elles soient d'origine naturelle ou synthétiques. Les vitamines hydrosolubles sont connues de l'homme du métier. Les vitamines hydrosolubles visées par l'invention sont, en particulier, les vitamines du groupe B, telle que la vitamine B1 (thiamine), la vitamine B2 (riboflavine), la vitamine B3 (niacine ou acide nicotinique), la vitamine B5 (acide pantothénique), la vitamine B6 (pyridoxine), la vitamine B8 (biotine ou vitamine H), la vitamine B9 (acide folique), la vitamine B12 (cobalamine), ainsi que la vitamine C (acide ascorbique). Les dérivés de ces vitamines hydrosolubles sont également visés par la présente invention et peuvent être leurs sels. De préférence, les dérivés des vitamines hydrosolubles sont des provitamines. Par « provitamines », on vise une substance pouvant être transformée en vitamine après ingestion par un animal. On citera, à titre d'exemple, le sel de calcium de la vitamine B5, à savoir le pantothénate de calcium, ou la cyanocobalamine, qui est une provitamine de la vitamine B12.

Les quantités de vitamines peuvent être déterminées par des méthodes bien connues de l'homme du métier. Par exemple, la quantité de vitamine B6 peut être déterminée suivant la norme NF EN 14164. La quantité de vitamine B9 peut, quant à elle être déterminée par analyse par chromatographie liquide de type HPLC (« High Pressure (Performance) Liquid Chromatoraphy ») ou UPLC (« Ultra Pressure (Performance) Liquid Chromatography ») avec une détection UV, RI (indice de réfraction) ou MS/MS (spectrométrie de masse en tandem). Ces méthodes de détermination, utilisées dans l'Exemple 2, sont préférées selon l'invention.

Dans toute la demande, lorsqu'aucune date n'est précisée pour un règlement, une norme ou une directive, il s'agit du règlement, de la norme ou de la directive en vigueur à la date de dépôt.

Le procédé selon l'invention permet de protéger les vitamines hydrosolubles présentes dans la composition, lors du traitement thermique. Par « protéger », on entend que le procédé selon l'invention permet d'éviter ou de limiter la dégradation des vitamines hydrosolubles présentes dans la composition, notamment au cours de sa granulation, telle qu'une granulation par extrusion. La présence de chitine dans la composition permet de protéger les vitamines hydrosolubles lors de ce traitement thermique.

La chitine est généralement considérée comme un composé difficile à digérer par les animaux, en particulier les poissons. La chitine est d'ailleurs souvent invoquée comme motif justifiant les résultats mitigés en termes de croissance des poissons nourris avec des farines d'insectes. Pourtant, comme cela est démontré à l'Exemple 4, la présence de chitine dans des aliments aquacoles n'a aucun impact négatif sur la croissance des poissons.

Avantageusement, la composition présente un taux d'humidité résiduel compris entre 2 et 15 %, de préférence entre 5 et 10%, plus préférentiellement entre 6 et 8%. Ce taux d'humidité peut par exemple être déterminé selon la méthode issue du règlement CE 152/2009 du 27-01-2009 (103 °C/ 4 h).

Selon un premier mode de réalisation du procédé selon l'invention, la composition comprend au moins 0,05% en poids de vitamines du groupe B, du groupe C et/ou leurs dérivés, par rapport au poids total de la composition, de préférence au moins 0,1% en poids.

Selon un second mode de réalisation du procédé selon l'invention, la composition comprend au moins 0,004% en poids de vitamines B6 et/ou B9 et/ou leurs dérivés par rapport au poids total de la composition, de préférence au moins 0,005% en poids, plus préférentiellement au moins 0,006% en poids.

Quel que soit le mode de réalisation du procédé selon l'invention, la composition comprend au moins 0,8% en poids de chitine, par rapport au poids total de la composition. En effet, l'effet protecteur des vitamines hydrosolubles est observable dès que la composition comporte au moins 0,8% en poids de chitine, par rapport au poids total de la composition.

Avantageusement, la composition comprend au moins 1,5% en poids de chitine par rapport au poids total de la composition, de préférence au moins 2% en poids de chitine par rapport au poids total de la composition, plus préférentiellement au moins 2,4% en poids de chitine par rapport au poids total de la composition.

Lorsque la composition comprend au moins 1,5% de chitine, on note une augmentation de l'effet protecteur sur les vitamines hydrosolubles, en particulier les vitamines B6 et/ou B9. Cet effet est encore supérieur lorsque la composition comprend au moins 2% de chitine, de préférence 2,4% de chitine. Ces effets sont plus amplement décrits dans l'Exemple 2 ci-après.

Dans le procédé selon l'invention, le traitement thermique est mis en œuvre à une température supérieure ou égale à 90°C.

De préférence, le traitement thermique est mis en œuvre à une température supérieure ou égale à 100 °C, plus préférentiellement comprise entre 115 et 145°C.

Le traitement thermique peut également être réalisé sous pression, ladite pression étant comprise entre 5 et 50 bars.

Avantageusement, le procédé selon l'invention est une granulation.

Dans le cadre de la présente demande, par « granulation », on vise un procédé d'assemblage de particules.

Par « granulé », on vise donc un produit résultant d'un procédé d'assemblage de particules.

De manière préférée, le procédé selon l'invention est une extrusion. Plus particulièrement, le procédé peut être une granulation par extrusion.

Selon un mode de réalisation préféré de l'invention, le procédé de granulation par extrusion comprend les étapes suivantes :

- le broyage d'une composition comprenant au moins 0,8% en poids de chitine et au moins 0,005% en poids de vitamines hydrosolubles, par rapport au poids total de la composition, pour obtenir une poudre;
- l'ajout de la poudre obtenue dans un extrudeur;
- le traitement de la poudre par l'extrudeur à une température comprise entre 90 et 250°C, et à une énergie spécifique mécanique (EMS) comprise entre 25 et 100 Wh/kg; et
- le séchage desdits granulés.

Par « broyage », on entend toute étape visant à obtenir une poudre.

De préférence, les particules formant la poudre obtenue à l'issue du broyage ont une taille comprise entre 10 et 850 μm, plus préférentiellement entre 100 et 500 μm, encore plus préférentiellement entre 150 et 350 μm.

Par extrudeur, on vise tout type d'extrudeur. De préférence, l'extrudeur est un extrudeur mono-vis ou bi-vis.

Suite à l'ajout de la poudre dans l'extrudeur et avant le traitement thermique, la poudre peut, optionnellement, être soumise à un prétraitement thermique à une température comprise 25 et 50°C. Cette étape de prétraitement thermique peut se faire dans un compartiment particulier de l'extrudeur, généralement appelé préconditionneur.

De préférence, l'énergie spécifique mécanique est comprise entre 35 et 85 Wh/kg, préférentiellement entre 50 et 70 Wh/kg.

Le procédé d'extrusion selon l'invention peut également comporter une étape d'enrobage. Par « enrobage », on vise toute étape d'application de composés, et en particulier de composés de type liquide, sur le granulé. Généralement, l'étape d'enrobage se fait par pulvérisation, par exemple à l'aide d'une enrobeuse, de ces composés sur la surface du granulé, par exemple avant l'étape de séchage.

Avantageusement, les composés incorporés sont des huiles, telles que des huiles végétales comme les huiles oléagineuses (colza), ou animales (poisson).

De préférence, la chitine présente dans la composition mise en œuvre dans le procédé de granulation par extrusion selon l'invention résulte de l'introduction d'une farine d'arthropodes, de céphalopodes (comme le calmar), de gastéropodes, d'annélides, et/ou de champignons dans ladite composition. Plus préférentiellement, la chitine résulte de l'introduction d'une farine d'insectes, de crustacés, de calmar et/ou de champignons, encore plus préférentiellement d'une farine d'insectes et/ou de crustacés.

Encore plus préférentiellement, la chitine présente dans la composition mise en œuvre dans le procédé de granulation par extrusion selon l'invention résulte de l'introduction d'une farine d'insectes et optionnellement d'une farine de crustacés.

De préférence, la ou les farine(s) représente(nt) entre 10 et 75%, de préférence entre 20 et 60%, plus préférentiellement entre 25 et 45% en poids sec du poids total de la composition mise en œuvre dans le procédé de granulation par extrusion selon l'invention.

Lorsque de la farine d'insectes est mise en œuvre, celle-ci représente avantageusement entre 7 et 50%, de préférence entre 10 et 40%, encore plus préférentiellement entre 25 et 35% en poids sec du poids total de la composition mise en œuvre dans le procédé de granulation par extrusion selon l'invention.

De préférence, la farine d'insectes est telle que décrite ci-avant.

La composition mise en oeuvre dans le procédé de granulation par extrusion selon l'invention pourra comporter en outre un ou plusieurs ingrédients choisis dans le groupe constitué par :

- la farine de poisson,
- du gluten, tel que du gluten de blé ou de maïs,
- une farine végétale, telle qu'une farine de plante(s) oléagineuse(s) ou protéagineuse(s), comme la farine de soja ou de pois, ou telle qu'une farine de céréales, comme la farine de blé,
- des huiles, telles que des huiles végétales, comme les huiles oléagineuses (colza), ou animales (poisson),
- des minéraux,
- des acides aminés tels que de la méthionine,
- des vitamines autres que des vitamines hydrosolubles.

Toutefois, et comme indiqué ci-avant, les huiles peuvent être ajoutées après extrusion, lors d'une étape d'enrobage.

Toutes les caractéristiques préférées du procédé de traitement thermique selon l'invention, telles que décrites ci-avant, peuvent également s'appliquer au présent mode de réalisation préféré de l'invention.

L'invention vise également un second procédé d'extrusion d'une composition (ne comportant pas ou peu de chitine) comportant les étapes suivantes :

- ajout d'au moins 0,8% de chitine à la composition, le pourcentage en poids étant donné sur le poids total de la composition, et
- extrusion de la composition.

En effet, il a été observé que l'ajout de chitine à une composition permettait de diminuer l'EMS nécessaire pour effectuer l'extrusion. L'invention concerne donc également l'utilisation de chitine pour diminuer l'EMS nécessaire à une extrusion.

La divulgation concerne également un granulé susceptible d'être obtenu par le procédé de granulation par extrusion selon l'invention.

L'invention vise, en outre, un granulé comprenant au moins 0,6% de chitine résultant de l'introduction d'une farine d'insectes, de crustacés, de calmar et/ou de champignons, et au moins 0,004% en poids de vitamines hydrosolubles, par rapport au poids total du granulé et ayant une densité apparente après séchage comprise entre 400 et 650 g/L.

De préférence, le granulé comprend au moins 1% en poids de chitine par rapport au poids total de granulé, plus préférentiellement au moins 1,2% en poids de chitine par rapport au poids total de granulé, encore plus préférentiellement au moins 2% en poids de chitine par rapport au poids total de granulé.

Selon un premier aspect, le granulé comprend au moins 0,04% en poids de vitamines du groupe B, du groupe C et/ou leurs dérivés, par rapport au poids total de granulé, de préférence au moins 0,08% en poids de vitamines du groupe B, du groupe C et/ou leurs dérivés, par rapport au poids total de granulé.

Selon un second aspect, le granulé comporte au moins 0,004% en poids de vitamines B6 et/ou B9, par rapport au poids total de granulé, de préférence au moins 0,005% en poids de vitamines B6 et/ou B9, par rapport au poids total de granulé.

De préférence, le granulé aura une taille comprise entre 1 et 10 mm, préférentiellement entre 1,5 et 5 mm, encore plus préférentiellement entre 2 et 4 mm.

La chitine présente dans le granulé résulte de l'introduction d'une farine d'insectes, de crustacés, de calmar et/ou de champignons, préférentiellement d'une farine d'insectes et/ou de crustacés.

Encore plus préférentiellement, la chitine présente dans le granulé résulte de l'introduction d'une farine d'insectes et optionnellement d'une farine de crustacés.

Avantageusement, le granulé comprend entre 6 et 75%, de préférence entre 15 et 55%, plus préférentiellement entre 25 et 45% en poids sec de farine(s), par rapport au poids total du granulé.

De préférence, le granulé comprend entre 6 et 50%, de préférence entre 10 et 40%, encore plus préférentiellement entre 20 et 30% en poids sec de farine d'insectes, par rapport au poids total du granulé.

De préférence, la farine d'insectes est telle que décrite ci-avant.

Le granulé pourra comporter en outre un ou plusieurs ingrédients choisis dans le groupe constitué par :

- la farine de poisson,
- du gluten, tel que du gluten de blé ou de maïs,
- une farine végétale, telle qu'une farine de plante(s) oléagineuse(s) ou protéagineuse(s), comme la farine de soja ou de pois, ou telle qu'une farine de céréales, comme la farine de blé,
- des huiles, telles que des huiles végétales, comme les huiles oléagineuses (colza), ou animales (poisson),
- des minéraux,
- des acides aminés tels que de la méthionine,
- des vitamines autres que des vitamines hydrosolubles.

Avantageusement, le granulé est obtenu par extrusion et présente les caractéristiques d'un granulé obtenu par extrusion.

En particulier, le granulé selon l'invention a une densité apparente après séchage comprise entre 400 et 650 g/L, avantageusement entre 450 et 600 g/L, de préférence entre 400 et 550 g/L, plus préférentiellement entre 400 et 550 g/L, encore plus préférentiellement entre 400 et 500 g/L.

De préférence, un granulé comportant l'ensemble des ingrédients ci-avant (la farine de poisson étant optionnelle) est particulièrement adapté pour l'alimentation des poissons.

L'invention concerne également l'utilisation d'un granulé selon l'invention, dans l'alimentation humaine ou animale.

Avantageusement, le granulé selon l'invention est utilisé dans l'alimentation des animaux de compagnie tels que les chiens, les chats, les oiseaux, les poissons.

De préférence, le granulé selon l'invention est utilisé dans l'alimentation des volailles (poulet, dinde, tout type de gibier (par exemple, caille, faisan, outarde), l'alimentation des porcins, des ruminants (bovins, ovins, caprins, équins) ou des visons, plus préférentiellement dans l'aquaculture (poissons, crustacés, mollusques, coquillages).

Le granulé selon l'invention peut également être utilisé comme complément alimentaire.

L'invention concerne enfin l'utilisation de la chitine pour la protection des vitamines hydrosolubles lors d'un traitement thermique à une température supérieure ou égale à 90°C.

En particulier, la chitine est utilisée pour la protection, lors dudit traitement thermique, des vitamines du groupe B, du groupe C et/ou leurs dérivés, et plus particulièrement des vitamines B6 et/ou B9.

Avantageusement, la chitine résulte de l'introduction d'une farine d'arthropodes, de céphalopodes (comme le calmar), de gastéropodes, d'annélides, et/ou de champignons.

Plus préférentiellement, la chitine résulte de l'introduction d'une farine d'insectes, de crustacés, de calmar et/ou de champignons, encore plus préférentiellement d'une farine d'insectes et/ou de crustacés.

Encore plus préférentiellement, la chitine résulte de l'introduction d'une farine d'insectes et optionnellement d'une farine de crustacés.

La farine d'insectes est avantageusement telle que décrite ci-avant.

L'invention sera mieux comprise au vu des exemples qui suivent, donnés à titre illustratif, avec référence à :

- La Figure 1, qui comporte trois diagrammes représentant les quantités de vitamines A (Fig. 1A), B6 (Fig. 1B) et B9 (Fig. 1C) dans des granulés obtenus selon le procédé selon l'invention, et dans des granulés obtenus selon un procédé comparatif;
- La Figure 2, qui est un diagramme représentant le pourcentage de perte en vitamine A, B6 et B9 avec le procédé selon l'invention et avec le procédé comparatif;
- La Figure 3, qui est un diagramme représentant la densité massique de granulés obtenus selon le procédé selon l'invention et de granulés obtenus selon un procédé comparatif.

### Exemple 1 : Procédé selon l'invention et procédé comparatif

### 1. Matériel et méthodes

#### 1.1. Matériel

#### Ingrédients pour la préparation des compositions à granuler

- Farine d'insectes

Une farine d'insectes dégraissée mécaniquement est obtenue par le traitement des larves de Tenebrio molitor. La composition de cette farine est présentée dans le Tableau 1 ci-dessous.

|  | Unité | Farine d'insecte |
|---|---|---|
| Humidité | %* | 5,3 |
| Protéine | %* | 67,1 |
| Matière grasse | %* | 13,6 |
| Fibre | %* | 1,6 |
| Cendre | %* | 3,2 |
| Chitine | %* | 8,0 |
| Énergie | MJ/kg | 23,7 |
| * Les % sont exprimés en poids sec par rapport au poids total de farine. |

**Tableau 1 : Composition de la farine d'insectes utilisée dans l'Exemple 1**

- Autres ingrédients

- Farine de poisson LT70 (Farine de poisson péruvien LT70: 71% de protéines brutes (PB), 11% de lipides bruts (LB), EXALMAR, Pérou)
- Farine de krill (2 à 4% de chitine) (Farine de krill : 61% PB, 19% LB, Aker BioMarine Antarctic AS, Norvège)
- Farine de calmar (Super Prime sans viscères: 82% PB, 3.5% LB, Sopropêche, France)
- Concentré de protéines de soja (Soycomil P: 62% PB, 0,7% LB, ADM, Pays Bas)
- Gluten de blé (VITEN: 84,7% PB, 1,3% LB, ROQUETTE, France)
- Gluten de maïs (Farine de gluten de maïs: 61% PB, 6% LB, COPAM, Portugal)
- Farine de soja 48
- Pois entier
- Huile de poisson
- Huile de colza
- Pré-mélange de vitamines et minéraux (PREMIX Lda, Portugal)
- Lécithine de soja
- Gomme de guar
- Antioxydant
- Propionate de sodium
- Phosphate Mono Calcique
- DL-méthionine

**Machine pour le traitement thermique**

Le traitement thermique est réalisé par une extrudeuse à deux vis (CLEXTRAL BC45, France) avec un diamètre de vis de 55,5 mm et un débit maximal de l'ordre de 90-100 kg/h. L'extrudeuse a été équipée d'une filière de forme ronde de taille 1 mm et d'un massicot à haute vitesse pour découper les produits dans la taille de granule définie.

**Autres matériels**

Le mélange est effectué dans un mélangeur à double hélice (modèle 500L, TGC Extrusion, France).
Le broyage est réalisé dans un broyeur à marteaux micro-pulvérisateur (modèle SH1, Hosokawa-Alpine, Allemagne).
Le séchage est effectué à l'aide d'un séchoir à lit fluidisé vibrant (modèle DR100, TGC Extrusion, France).
L'enrobage est réalisé dans une enrobeuse (modèle PG-10VCLAB, Dinnisen, Pays-Bas).

**1.2. Méthodes**

**Préparation des compositions**

Trois compositions Y7,5, Y15 et Y25 ont été formulées, comprenant respectivement 7,5%, 15% et 25% en poids sec de farine d'insectes sur le poids sec total de la composition. Ces trois compositions ont été mises en œuvre dans des procédés selon l'invention. Deux autres compositions, CTRL et Y5, ont également été formulées pour une mise en œuvre dans des procédés comparatifs.
Certains ajustements sur la formulation des compositions ont été faits pour maintenir les conditions isoazotées (protéine brute, 48,5% en poids sec sur le poids total de la matière sèche (MS)), isolipidiques (22,7% en poids sec sur le poids total de la MS) et isoénergétiques (énergie brute, 23,2 MJ/ kg de MS).
Tous les ingrédients (à l'exception des huiles de poisson et de colza) ont été pesés et mélangés selon les différentes formulations dans le mélangeur à double hélice.
Les différents mélanges obtenus ont été broyés dans le broyeur à marteaux micro-pulvérisateur pour obtenir les compositions CTRL, Y5, Y7,5, Y15 et Y20, sous forme de poudre dont les particules ont une taille inférieure à 250 microns.
60 kg de chaque composition ont été préparés.
Les formulations des compositions sont résumées dans le Tableau 2 ci-dessous.

| Ingrédients en %* : | CTRL | Y5 | Y7.5 | Y15 | Y25 |
|---|---|---|---|---|---|
| Farine de poisson LT70 | 25,00 | 20,00 | 17,50 | 10,00 | 0,00 |
| Farine de krill | 3,00 | 3,00 | 3,00 | 3,00 | 3,00 |
| Farine de calmar | 5,00 | 5,00 | 5,00 | 5,00 | 5,00 |
| Farine d'insecte | | 5,00 | 7,50 | 15,00 | 25,00 |
| Concentré de protéines de soja | 14,00 | 14,00 | 14,00 | 14,00 | 14,00 |
| Gluten de blé | 9,05 | 9,25 | 9,40 | 9,65 | 10,10 |
| Gluten de maïs | 8,20 | 8,20 | 8,20 | 8,20 | 8,20 |
| Farine de soja 48 | 7,50 | 7,50 | 7,50 | 7,50 | 7,50 |
| Pois entier | 6,15 | 5,75 | 5,40 | 4,75 | 3,70 |
| Huile de poisson | 11,50 | 11,50 | 11,50 | 11,50 | 11,50 |
| Huile de colza | 6,00 | 5,80 | 5,70 | 5,40 | 5,00 |
| Pré-mélange de vitamines et minéraux | 1,50 | 1,50 | 1,50 | 1,50 | 1,50 |
| Lécithine de soja | 1,00 | 1,00 | 1,00 | 1,00 | 1,00 |
| Gomme de guar | 0,20 | 0,20 | 0,20 | 0,20 | 0,20 |
| Antioxydant | 0,20 | 0,20 | 0,20 | 0,20 | 0,20 |
| Propionate de sodium | 0,10 | 0,10 | 0,10 | 0,10 | 0,10 |
| Phosphate Mono Calcique | 1,30 | 1,70 | 2,00 | 2,60 | 3,50 |
| DL-méthionine | 0,30 | 0,30 | 0,30 | 0,40 | 0,50 |
| | | | | | |
| Matière sèche (MS), %* | 93,4 ± 0,0 | 93,1 ± 0,0 | 93, ± 0,1 | 95,0 ± 0,0 | 93,2 ± 0,0 |
| Protéine brute, % MS** | 48,5 ± 0,0 | 48,5 ± 0,1 | 48,5 ± 0,0 | 48,5 ± 0,0 | 48,5 ± 0,1 |
| Matières grasses brutes,% MS** | 22,7 ± 0,2 | 22,7 ± 0,1 | 22,6 ± 0,2 | 22,7 ± 0,2 | 22,7 ± 0,2 |
| Cendre, % MS** | 9,4 ± 0,0 | 8,8 ± 0,0 | 8,7 ± 0,1 | 8,1 ± 0,0 | 7,4 ± 0,0 |
| Chitine, % MS** | 0,06 | 0,46 | 0,66 | 1,26 | 2,06 |
| Énergie brute, MJ/kq de MS | 23,2 ± 0,2 | 23,2 ± 0,0 | 23,2 ± 0,0 | 23,2 ± 0,1 | 23,2 ± 0,1 |
| Total | 100 | 100 | 100 | 100 | 100 |
| Total sans huiles (poisson et colza) | 82,5 | 82,7 | 82,8 | 83,1 | 83,5 |
| Chitine, % MS sans huiles (poisson et colza) | 0,07 | 0,56 | 0,80 | 1,52 | 2,47 |
| * % de matière sèche par rapport au poids total de la composition **** % en poids sec par rapport au poids total de la matière sèche | | | | | |

**Tableau 2 : Résumé des compositions préparées.**

Le pré-mélange de vitamines et minéraux mentionné dans le Tableau 2 comporte :

- 0,3% en poids de vitamine B1 (thiamine),

- 0,3% en poids de vitamine B2 (riboflavine),

- 0,2% en poids de vitamine B6 (pyridoxine),

- 0,001% en poids de provitamine de la vitamine B12 (cyanocobalamine),

- 2% en poids de vitamine B3 (acide nicotinique ou niacine),

- 0,15% en poids de vitamine B9 (acide folique),

- 2% de vitamine C (acide ascorbique),

- 0,03% en poids de vitamine B8 (biotine ou vitamine H), et

- 1% en poids de provitamine de la vitamine B5 (pantothénate de calcium),

les pourcentages en poids étant indiqués par rapport au poids total du pré-mélange de vitamines et minéraux.

Les compositions CTRL, Y5, Y7.5, Y15 et Y25 comprennent donc environ 0,11% en poids de vitamines hydrosolubles et/ou leurs dérivés dont 0,006% en poids de vitamines B6 et B9, par rapport au poids total sans huiles (poisson et colza) de la composition.

### Détermination de la quantité de chitine

Les fibres alimentaires de la farine d'insectes sont essentiellement composées de chitine, cette dernière a donc été dosée suivant la méthode ADAC 991.43. Les valeurs ainsi obtenues sont par conséquent légèrement surestimées.

### Traitement thermique des compositions

Les compositions CTRL, Y5, Y7,5, Y15 et Y25 ont ensuite été extrudées à une taille de granule de 3,0 mm.

Traitement thermique

La température du traitement thermique réalisée lors de l'extrusion est indiquée dans le Tableau 3 ci-dessous, pour chaque composition.

Tout au long de l'extrusion des différentes compositions, les paramètres de fonctionnement ont été enregistrés et ont permis le calcul de l'énergie mécanique spécifique (EMS), en Watt heure/kg (Wh/kg).

| Régime | Temp. Fût 1 (°C) | Temp. Fût 2 (°C) | Taux d'approvisionnement (kg/h) | Vitesse de la vis (tr/min) | Niveau de l'eau (0-10) | Ampérage (A) | EMS (Wh/kg) |
|---|---|---|---|---|---|---|---|
| CTRL | 32-33 | 120-123 | 87 | 234 | 7,0 | 15 | **66,0** |
| Y5 | 32-33 | 120-122 | 90 | 240 | 7,0 | 15 | **65,5** |
| Y7.5 | 32-33 | 119-121 | 90 | 245 | 7,0 | 14 | **62,4** |
| Y15 | 32-33 | 119-122 | 93 | 252 | 6,5 | 13 | **57,7** |
| Y25 | 32-33 | 120-123 | 95 | 257 | 6,0 | 13 | **57,6** |

**Tableau 3 : Conditions de l'extrusion et EMS dans l'Exemple 1.**

Par « Fût 1 », on vise un préconditionneur, où le mélange issu du mélangeur double hélice est mis à température.

Par « Fût 2 », on vise un conditionneur : c'est l'étape de traitement thermique et de montée en pression qui a lieu dans l'extrudeur.

Le niveau de l'eau est une indication de l'eau ajoutée lors du chauffage par la vapeur et par conséquent la quantité ajoutée peut varier selon les ingrédients. Elle est ajustée par séchage à la fin du procédé.

L'EMS a été calculée comme suit :

$$EMS\,(W\,h/kg) = \frac{U \times I \times \cos\Phi \, \dfrac{ESS\,SS}{Max\,SS}}{Qs}$$

Où :

U : tension de fonctionnement du moteur (U = 460 V).

I: courant alimentant le moteur (A).

cos φ : rendement théorique du moteur de l'extrudeuse (cos φ = 0,95).

ESS SS : vitesse d'essai (tr/min) des vis en rotation.

Max SS : vitesse maximum (267 tr/min) des vis en rotation.

Qs : débit d'entrée de la composition (kg/h).

### Séchage

Suite à l'extrusion, les extrudats CTRL, Y5, Y7,5, Y15 et Y25 ont été séchés dans le séchoir à lit fluidisé vibrant.

### Enrobage

Après refroidissement des granulés Y7,5, Y15 et Y25, les huiles de poisson et de colza décrites dans le Tableau 2 ont été ajoutées par enrobage sous vide à l'aide de l'enrobeuse.

### 2. Conclusion

Au cours du processus d'extrusion, des doses croissantes de farine d'insectes nécessitent une réduction de l'eau ajoutée et une augmentation des taux d'alimentation de la mêlée et de la vitesse de vis, ce qui a entraîné une réduction de l'énergie mécanique spécifique (EMS).

### Exemple 2 : Evaluation des vitamines hydrosolubles dans des granulés obtenus selon le procédé selon l'invention et selon des procédés comparatifs

Afin de déterminer l'effet sur la conservation des vitamines hydrosolubles, des échantillons des compositions CTRL, Y5, Y7,5, Y15 et Y25 avant extrusion, des extrudats CTRL, Y5, Y7,5, Y15 et Y25 (avant séchage), et des granulés séchés CTRL, Y5, Y7,5, Y15 et Y25 (sans enrobage/revêtement) ont été prélevés pour l'analyse de la teneur en vitamine A, B6 et B9.

### 1. Matériel et méthodes

La quantité de vitamine B6 a été déterminée suivant la norme NF EN 14164.

La quantité de vitamine B9 a été déterminée par analyse par chromatographie liquide de type HPLC ou UPLC avec une détection UV, RI ou MS/MS.

Les résultats de ces analyses sont présents dans le Tableau 4 ci-dessous.

| | Composition avant extrusion | Extrudat | Granulé sec (sans revêtement) |
|---|---|---|---|
| **Vitamine A (UI/kg)** | | | |
| CTRL | 29750 ± 1219 | 28300 ± 1395 | 28808 ±1303 |
| Y5 | 31395 ± 1445 | 29382 ± 325 | 30463 ± 648 |
| Y7,5 | 30625 ± 601 | 31183 ±550 | 30129 ±800 |
| Y15 | 31331 ± 761 | 29857 ± 1762 | 30287 ± 1150 |
| Y25 | 30210 ± 1454 | 28290 ± 1018 | 29699 ± 752 |
| **Vitamine B6 (mg/kg*)** | | | |
| CTRL | 23,3 ± 0,6 | 21,7 ± 0,1 | 20,7 ± 0,9 |
| Y5 | 24,1 ± 0,6 | 22,1 ± 0,1 | 21,3 ± 0,9 |
| Y7,5 | 24,8 ± 0,5 | 23,5 ± 0,4 | 22,6 ± 0,4 |
| Y15 | 26,3 ± 0,7 | 27,5 ± 0,6 | 25,5 ± 0,6 |
| Y25 | 26,4 ± 0,0 | 27,6 ± 1,8 | 26,8 ± 1,0 |

(suite)

| Vitamine B9 (mg/kg*) | | | |
|---|---|---|---|
| CTRL | 16,7 ± 0,7 | 8,3 ± 0,4 | 13,9 ± 0,8 |
| Y5 | 16,1 ± 1,0 | 10,6 ± 1,4 | 17,2 ± 0,7 |
| Y7,5 | 16,8 ± 1,3 | 12,0 ± 1,7 | 16,8 ± 1,5 |
| Y15 | 17,7 ± 0,2 | 13,4 ± 0,7 | 17,0 ± 0,1 |
| Y25 | 16,6 ± 1,2 | 13,6 ± 1,0 | 17,2 ± 0,2 |
| * Poids indiqués en mg/kg de composition | | | |

**Tableau 4 : Quantités de vitamine A, B6 et B9 à divers stades du procédé de granulation**

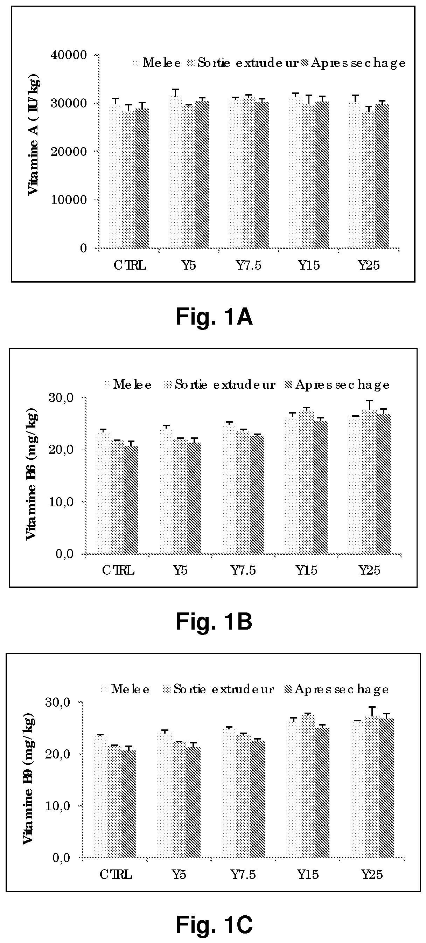

Les résultats du Tableau 4 sont représentés à la Figure 1.

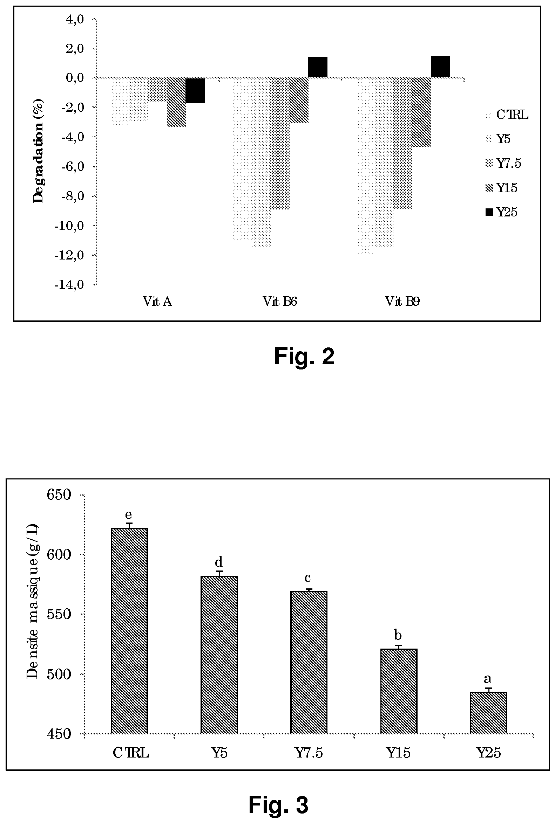

La Figure 2 montre le pourcentage de perte en vitamine A, B6 et B9 lors du procédé de granulation pour chaque composition CTRL, Y5, Y7,5, Y15 et Y25.

Les résultats montrent que :

- Les pertes de vitamine A (liposoluble) au cours de la granulation étaient faibles (2-3%) et la vitamine A est peu affectée par la présence de chitine.
- Dans un régime CTRL, les pertes de vitamines B6 et B9 (hydrosolubles) pendant l'extrusion ont varié de 11 à 12%. Cependant, l'inclusion de farine contenant de la chitine à 15 et 25% tendait à réduire significativement les pertes de traitement de ces deux vitamines. Cet effet de préservation des vitamines hydrosolubles est donc observable dès qu'un taux de chitine supérieur ou égal à 0,8% est atteint dans la composition subissant le traitement thermique, de préférence supérieur ou égal à 1,5%.

**Exemple 3 : Evaluation des critères de qualité des granulés**

**1. Matériel et méthodes**

La qualité des granulés CTRL, Y5, Y7,5, Y15 et Y25 tels que préparés à l'Exemple 1 a été évaluée.

**1.1. Teneur en humidité**

Le niveau d'humidité de l'extrudat (à la sortie de l'extrudeuse et avant le séchage) et des granulés (une fois séchés) a été mesuré, en déterminant la perte de poids après séchage de 10 grammes d'échantillon (fait en trois exemplaires) à 105°C pendant 24 h.

**1.2. Activité de l'eau**

L'activité de l'eau (aw) représente la pression de vapeur d'eau d'un produit divisée par la pression de vapeur d'une eau pure à la même température. L'activité de l'eau est couramment utilisée comme critère pour caractériser la durée de conservation des produits, car en dessous de certains niveaux, elle inhibe la croissance des bactéries et des moisissures. L'activité de l'eau a été mesurée dans des échantillons en triple exemplaires (2,5 g) d'aliments à l'aide de AquaLab LITE (DECAGON, USA).

**1.3. Densité apparente**

La densité apparente des aliments extrudés a été déterminée, en triple exemplaire, en remplissant un bêcher volumétrique en plastique pré-pesé (volume connu de 1 L) avec des granulés. L'excès de granulés au sommet du bêcher en plastique a été gratté doucement sur le bord. Une précaution a été accordée pour éviter l'entaillage de l'échantillon. Le bêcher en plastique a été pesé et la densité apparente exprimée en masse de l'échantillon (g) par unité de volume (L).

**1.4. Traitement statistique des données**

Les données ont été analysées par une ANOVA unidirectionnelle. Le cas échéant, les moyennes ont été comparées par le test de Newman-Keuls. La signification statistique a été testée à P <0,05. Tous les tests statistiques ont été effectués en utilisant le logiciel SPSS (v21, IBM, USA).

## 2. Résultats

Les résultats relatifs à la qualité des différents granulés sont présentés dans le Tableau 5 ci-après et en Figure 3.

| Régime | Densité apparente après séchage g/L | Humidité à la sortie de l'extrudeuse % | Humidité après séchage % | Activité de l'eau après séchage |
|---|---|---|---|---|
| CTRL | 622 ± 5[e] | 21,8 ± 0,3 | 6,7 ± 0,1 | 0,528 ± 0,006 |
| Y5 | 582 ± 4[d] | 22,2 ± 0,6 | 6,9 ± 0,1 | 0,532 ± 0,017 |
| Y7,5 | 569 ± 2[c] | 22,2 ± 0,6 | 6,7 ± 0,1 | 0,535 ± 0,009 |
| Y15 | 521 ± 3[b] | 21,8 ± 0,6 | 6,6 ± 0,2 | 0,532 ± 0,003 |
| Y25 | 485 ± 4[a] | 21,6 ± 0,9 | 6,8 ± 0,1 | 0,531 ± 0,004 |

Les valeurs sont des moyennes ± écart type (n = 3). Différentes lettres en exposant dans une colonne indiquent une différence significative (P <0,05).

**Tableau 5 : Densité apparente, taux d'humidité et activité de l'eau des aliments expérimentaux**

## 3. Conclusion

La densité apparente des granulés extrudés a varié considérablement, de 622 à 485 g/L. Une réduction significative de la densité apparente (P <0,05) a été étroitement associée à l'augmentation des doses croissantes de la farine d'insectes.

Quel que soit le niveau d'inclusion, la farine d'insectes n'a pas affecté la teneur en humidité (mesurée à la fois avant et après le séchage) et l'activité de l'eau des aliments extrudés.

Il est intéressant de souligner que dans le but de maintenir des conditions isoazotées et isolipidiques entre les régimes, outre le remplacement direct de farine de poisson par la farine d'insectes, les régimes riches en farine d'insectes ont une légère augmentation de gluten de blé (de 9,05% en CTRL à 10,10% en Y25) et une réduction de pois entiers (de 6,15% en CTRL à 3,70% en Y25). À la fois le gluten de froment et l'amidon provenant de pois entiers sont des éléments connus pour affecter l'expansion et la structure physique des granulés.

## Exemple 4 : Impact de la chitine sur la croissance des poissons

Le régime CTRL décrit à l'Exemple 1 a été formulé avec des ingrédients pratiques pour répondre aux besoins nutritionnels connus des truites arc-en-ciel juvéniles. Ce régime CTRL est composé de 25% de farine de poisson, 8% d'autres sources protéiques d'origine marine (farine de calmar et farine de krill), tandis que les sources de protéines restantes étaient un concentré de protéine de soja, de gluten de blé et de gluten de maïs. Sur la base de cette formulation, les quatre régimes Y5, Y7,5, Y15 et Y25, également décrit à l'Exemple 1, ont été formulés, dans lesquels la farine de poisson a été remplacée par la farine d'insectes à des taux respectifs de 20, 30, 60 et 100%.

### 1. Matériel et méthodes

### 1.1. Matériel

### Les régimes alimentaires

Les 5 régimes sont constitués de granulés ayant les compositions CTRL, Y5, Y7,5, Y15 et Y25 respectivement, tels que préparés à l'Exemple 1.

Les niveaux de farine de calmar et de krill ont été maintenus constants parmi tous les régimes afin de garantir une haute palatabilité. Des ajustements mineurs sur la formulation des régimes testés ont été faits pour maintenir les conditions isoazotées (protéine brute, 48,5% de MS), isolipidiques (22,7% MS) et isoénergétiques (énergie brute, 23,2 MJ/ kg MS).

Les niveaux de supplémentation en méthionine et en phosphate monocalcique dans les régimes testés ont été ajustés pour correspondre à ceux trouvés dans l'alimentation CTRL.

Pendant toute la durée de l'essai, les aliments expérimentés ont été stockés à température ambiante, mais dans un emplacement frais et aéré.

## Les poissons

Des groupes en trois exemplaires de 35 truites arc en ciel (*Oncorhynchus mykiss*), avec un poids corporel initial (PCI) de 5,01± 0,1 g ont été nourris avec l'un des cinq régimes expérimentaux pendant 90 jours. Les poissons ont grandi dans des réservoirs circulaires en fibre de verre (volume : 250 L) alimentés en eau douce à écoulement continu, à des températures comprises entre 14,1 ± 0,3°C et des niveaux d'oxygène dissous au-dessus de 7,4 mg/L (Figure 1). Les poissons ont été soumis par des conditions d'été à des changements de photopériode naturelle (mai-juillet). Les poissons ont été nourris à satiété apparente, à la main, trois fois par jour (9h00, 14h00 et 18h00) en semaine et deux fois par jour les week-ends (10h00 et 16h00), avec le plus grand soin pour éviter le gaspillage d'aliments. L'aliment distribué a été quantifié tout au long de l'étude. Des poissons anesthésiés ont été pesés individuellement au début et à la fin de l'étude et le groupe a été pesé au jour 28 et au jour 60. Au début, 15 poissons du même stock initial ont été échantillonnés et stockés à -20°C pour une analyse ultérieure de la composition corporelle intégrale. Après 90 jours d'alimentation expérimentale, 6 poissons de chaque réservoir ont été échantillonnés dans le même but.

### 1.2. Méthodes

### Critère pour l'évaluation de la croissance et l'utilisation des nutriments

PCI (g): Poids corporel initial.
PCF (g): Poids corporel final.

Taux de croissance spécifique, TCS (% / jour) : (Ln PCF – Ln PCI) x 100/jours.

Indice de consommation, IC : ration alimentaire brute / prise de poids.

Apport alimentaire volontaire, AAV (%PC/jour) : (ration alimentaire brute / (PCI+PCF) / 2 / jours) x 100.

Coefficient d'efficacité protéique, CEP : prise de poids mouillé / apport en protéine brute.

### Analyse statistique

Les données sont présentées par la moyenne de trois répétitions ± l'écart type. Les données ont été soumises à une analyse de la variance à un facteur. Avant ANOVA, les valeurs exprimées en % ont été soumises à une transformation de la racine carrée arc sinus. La signification statistique a été testée à un niveau d'une probabilité de 0,05. Tous les tests statistiques ont été effectués en utilisant le logiciel IBM SPSS V21.

### 2. Résultats

### Performance de croissance

Les données sur les performances de croissance, la conversion alimentaire et l'efficacité protéique de la truite arc-en-ciel nourrie durant 90 jours avec les régimes expérimentaux sont reportées dans le Tableau 6. Aucune mortalité n'est survenue au cours de l'essai.

**EP 3 364 777 B1**

**Tableau 6 : Performances de croissance au jour 90**

| Régime | CTRL | Y5 | Y7.5 | Y15 | Y25 |
|---|---|---|---|---|---|
| PCI (g) | 5,0 ± 0,1 | 4,9 ± 0,1 | 5,0 ± 0,1 | 5,1 ± 0,1 | 5,1 ± 0,1 |
| PCF (g) | 42,9 ± 1,3 a | 45,2 ± 1,0 b | 49,0 ± 0,6 c | 51,0 ± 1,4 c | 55,9 ± 1,0 d |
| TCS, %/j | 2,39 ± 0,06[a] | 2,47 ± 0,02 [b] | 2,54 ± 0,03 [b] | 2,56 ± 0,05 [b] | 2,67 ± 0,04 [c] |
| IC | 0,93 ± 0,02 [b] | 0,83 ± 0,03 [a] | 0,80 ± 0,02 [a] | 0,79 ± 0,04 [a] | 0,79 ± 0,02 [a] |
| Apport alimentaire, %PCM/j | 1,63 ± 0,04 [b] | 1,48 ± 0,05 [a] | 1,45 ± 0,04 [a] | 1,44 ± 0,07 [a] | 1,47 ± 0,05 [a] |
| CEP | 2,38 ± 0,06 [a] | 2,68 ± 0,10 [b] | 2,76 ± 0,06 [b] | 2,80 ± 0,15 [b] | 2,74 ± 0,08 [b] |

Les valeurs sont les moyennes ± l'écart type (n=3).
Les valeurs au sein d'une rangée avec des exposants différents diffèrent de façon significative (P <0,05).

L'incorporation de doses croissantes de farine d'insectes (et donc de doses croissantes de chitine) avec une réduction concomitante de la farine de poisson a été progressivement liée à une augmentation significative du poids corporel du poisson. De plus, en comparaison avec le traitement CTRL, tous les régimes de repas d'insectes, ont conduit à une réduction significative de l'apport alimentaire et une augmentation significative des valeurs du CEP (P <0,05). L'introduction de chitine dans les granulés selon l'invention ne présente donc pas d'inconvénients quant à l'apport nutritionnel délivré à l'animal.

**Revendications**

1. Procédé comprenant un traitement thermique d'une composition à une température supérieure ou égale à 90°C, dans lequel la composition comprend au moins 0,8% en poids de chitine et au moins 0,005% en poids de vitamines hydrosolubles et/ou leurs dérivés, par rapport au poids total de la composition.

2. Procédé selon la revendication 1, dans lequel la composition comprend au moins 0,05% en poids de vitamines du groupe B, du groupe C et/ou leurs dérivés, par rapport au poids total de la composition.

3. Procédé selon la revendication 1, dans lequel la composition comprend au moins 0,004% en poids de vitamines B6 et/ou B9 et/ou leurs dérivés, par rapport au poids total de la composition.

4. Procédé selon l'une quelconque des revendications 1 à 3, dans lequel la composition comprend au moins 1,5% en poids de chitine par rapport au poids total de la composition.

5. Procédé selon l'une quelconque des revendications 1 à 4, dans lequel la température est supérieure ou égale à 100 °C.

6. Procédé selon l'une quelconque des revendications 1 à 5, dans lequel le traitement thermique est réalisé sous pression.

7. Procédé selon l'une quelconque des revendications 1 à 6, dans lequel le procédé est une granulation.

8. Procédé selon l'une quelconque des revendications 1 à 7, dans lequel le procédé est une extrusion.

9. Granulé comprenant au moins 0,6% de chitine résultant de l'introduction d'une farine d'insectes, de crustacés, de calmar et/ou de champignons, et au moins 0,004% en poids de vitamines hydrosolubles, par rapport au poids total du granulé, et ayant une densité apparente après séchage comprise entre 400 et 650 g/L.

10. Utilisation de la chitine pour la protection des vitamines hydrosolubles lors d'un traitement thermique à une température supérieure ou égale à 90°C.

**11.** Procédé selon l'une quelconque des revendications 1 à 8, utilisation selon la revendication 10, dans lesquels la chitine résulte de l'introduction d'une farine d'insectes, de crustacés, de calmar et/ou de champignons.

**12.** Utilisation d'un granulé selon la revendication 9, dans l'alimentation humaine ou animale.

**Patentansprüche**

**1.** Verfahren, welches eine thermische Behandlung einer Zusammensetzung bei einer Temperatur höher als oder gleich 90 °C umfasst, in welchem die Zusammensetzung mindestens 0,8 Gewichtsprozent Chitin und mindestens 0,005 Gewichtsprozent an wasserlöslichen Vitaminen und/oder deren Derivaten umfasst, bezogen auf das Gesamtgewicht der Zusammensetzung.

**2.** Verfahren nach Anspruch 1, in welchem die Zusammensetzung mindestens 0,05 Gewichtsprozent an Vitaminen der Gruppe B, der Gruppe C und/oder deren Derivaten umfasst, bezogen auf das Gesamtgewicht der Zusammensetzung.

**3.** Verfahren nach Anspruch 1, in welchem die Zusammensetzung mindestens 0,004 Gewichtsprozent an Vitamin B6 und/oder B9 und/oder deren Derivaten umfasst, bezogen auf das Gesamtgewicht der Zusammensetzung.

**4.** Verfahren nach einem der Ansprüche 1 bis 3, in welchem die Zusammensetzung mindestens 1,5 Gewichtsprozent Chitin umfasst, bezogen auf das Gesamtgewicht der Zusammensetzung.

**5.** Verfahren nach einem der Ansprüche 1 bis 4, in welchem die Temperatur höher als oder gleich 100 °C ist.

**6.** Verfahren nach einem der Ansprüche 1 bis 5, in welchem die thermische Behandlung unter Druck durchgeführt wird.

**7.** Verfahren nach einem der Ansprüche 1 bis 6, in welchem das Verfahren eine Granulierung ist.

**8.** Verfahren nach einem der Ansprüche 1 bis 7, in welchem das Verfahren eine Extrusion ist.

**9.** Granulat, welches mindestens 0,6 Gewichtsprozent Chitin, das aus der Zugabe eines Insekten-, Krustentier-, Kalmar- und/oder Pilz-Mehls stammt, und mindestens 0,004 Gewichtsprozent wasserlösliche Vitamine umfasst, bezogen auf das Gesamtgewicht des Granulats, und welches eine Schüttdichte nach Trocknung aufweist, die zwischen 400 und 650 g/L liegt.

**10.** Verwendung von Chitin zum Schutz wasserlöslicher Vitamine bei einer thermischen Behandlung bei einer Temperatur höher als oder gleich 90 °C.

**11.** Verfahren nach einem der Ansprüche 1 bis 8 oder Verwendung nach Anspruch 10, in welchen das Chitin aus der Zugabe eines Insekten-, Krustentier-, Kalmar- und/oder Pilz-Mehls stammt.

**12.** Verwendung eines Granulats nach Anspruch 9 in der menschlichen oder tierischen Ernährung.

**Claims**

**1.** Method comprising heat treatment of a composition at a temperature greater than or equal to 90°C, wherein the composition comprises at least 0.8% by weight of chitin and at least 0.005% by weight of water-soluble vitamins and/or their derivatives, relative to the total weight of the composition.

**2.** Method according to claim 1, wherein the composition comprises at least 0.05% by weight of vitamins of group B, of group C and/or their derivatives, relative to the total weight of the composition.

**3.** Method according to claim 1, wherein the composition comprises at least 0.004% by weight of vitamins B6 and/or B9 and/or their derivatives, relative to the total weight of the composition.

**4.** Method according to any of claims 1 to 3, wherein the composition comprises at least 1.5% by weight of chitin relative to the total weight of the composition.

5.  Method according to any of claims 1 to 4, wherein the temperature is greater than or equal to 100°C.

6.  Method according to any of claims 1 to 5, wherein the heat treatment is carried out under pressure.

7.  Method according to any of claims 1 to 6, wherein the method is granulation.

8.  Method according to any of claims 1 to 7, wherein the method is extrusion.

9.  Granule comprising at least 0.6% of chitin resulting from the introduction of a flour of insects, crustaceans, squid and/or fungi, and at least 0.004% by weight of water-soluble vitamins, relative to the total weight of the granule, and having a bulk density after drying of between 400 and 650 g/L.

10. Use of chitin for the protection of water-soluble vitamins during heat treatment at a temperature greater than or equal to 90°C.

11. Method according to any of claims 1 to 8, use according to claim 10, wherein the chitin results from the introduction of a flour of insects, crustaceans, squid and/or fungi.

12. Use of a granule according to claim 9, in human or animal nutrition.

**Fig. 1A**

**Fig. 1B**

**Fig. 1C**

Fig. 2

Fig. 3

## RÉFÉRENCES CITÉES DANS LA DESCRIPTION

### Documents brevets cités dans la description

- CN 104382059
- CN 101779723